# EUROPEAN PATENT SPECIFICATION

(11) **EP 2 600 862 B1**
(45) Date of publication and mention of the grant of the patent: **20.04.2016**
(21) Application number: 11815379.0
(22) Date of filing: 05.08.2011
(51) Int. Cl.: A61K 31/353, A61K 31/4184, A61K 31/439, A61P 25/00, A61P 43/00

(54) **INHIBITORS OF ERK FOR DEVELOPMENTAL DISORDERS OF NEURONAL CONNECTIVITY**
ERK-HEMMER FÜR ENTWICKLUNGSSTÖRUNGEN DER NEURONALEN KONNEKTIVITÄT
INHIBITEURS DE L'ERK DESTINÉS À TRAITER DES TROUBLES DU DÉVELOPPEMENT DE LA CONNECTIVITÉ NEURONALE

(30) Priority: 21.10.2010 US 405369 P; 05.08.2010 US 370854 P
(43) Date of publication of application: 12.06.2013
(73) Proprietor: Case Western Reserve University, Cleveland, OH 44106 (US)
(72) Inventor: SMITH, Mark, A., Chagrin Falls OH 44023 (US); SNAPE, Michael, Cleveland OH 44106 (US)
(74) Representative: Maiwald Patentanwalts GmbH
(86) International application number: PCT/US2011/046773
(87) International publication number: WO 2012/019113

(56) References cited:
- WO-A1-2006/134469
- WO-A1-2011/016799
- US-A1- 2004 087 583
- US-A1- 2007 105 859
- US-A1- 2008 103 105
- US-A1- 2010 197 676
- ERIC HOLLANDER ET AL: "A Placebo Controlled Crossover Trial of Liquid Fluoxetine on Repetitive Behaviors in Childhood and Adolescent Autism", NEUROPSYCHOPHARMACOLOGY, vol. 30, no. 3, 15 December 2004 (2004-12-15), pages 582-589, XP055092698, ISSN: 0893-133X, DOI: 10.1038/sj.npp.1300627
- G ROBERT DELONG ET AL: "Effects of fluoxetine treatment in young children with idiopathic autism", DEVELOPMENTAL MEDICINE AND CHILD NEUROLOGY, vol. 40, no. 8, 12 August 1998 (1998-08-12), pages 551-562, XP055092706, GB ISSN: 0012-1622, DOI: 10.1111/j.1469-8749.1998.tb15414.x
- R. J. HAGERMAN ET AL: "Advances in the Treatment of Fragile X Syndrome", PEDIATRICS, vol. 123, no. 1, 1 January 2009 (2009-01-01), pages 378-390, XP055029528, ISSN: 0031-4005, DOI: 10.1542/peds.2008-0317
- HAGERMAN R J ET AL: "A survey of fluoxetine therapy in fragile X syndrome", DEVELOPMENTAL BRAIN DYSFUNCTION 1994 CH, vol. 7, no. 2-3, 1994, pages 155-164, XP009175232, ISSN: 1019-5815
- S.-C. CHUANG: "Prolonged Epileptiform Discharges Induced by Altered Group I Metabotropic Glutamate Receptor-Mediated Synaptic Responses in Hippocampal Slices of a Fragile X Mouse Model", JOURNAL OF NEUROSCIENCE, vol. 25, no. 35, 31 August 2005 (2005-08-31), pages 8048-8055, XP55093890, ISSN: 0270-6474, DOI: 10.1523/JNEUROSCI.1777-05.2005
- EMMANUEL VALJENT ET AL: "Addictive and non-addictive drugs induce distinct and specific patterns of ERK activation in mouse brain", EUROPEAN JOURNAL OF NEUROSCIENCE, vol. 19, no. 7, 1 April 2004 (2004-04-01), pages 1826-1836, XP055093956, ISSN: 0953-816X, DOI: 10.1111/j.1460-9568.2004.03278.x

## Description

This application claims priority from U.S. Provisional Application Nos. 61/370,854, filed August 5, 2010 and 61/405,369, filed October 21, 2010.

### FIELD OF THE INVENTION

This application relates to the treatment of childhood onset disorders in which neuronal connectivity is compromised, such that normal brain development and cognitive function are compromised.

### BACKGROUND

Developmental disorders, such as Fragile X Syndrome and autism, are characterized by an onset in early childhood, persistence throughout life, and compromised brain function. An affected individual will be affected with respect to multiple abilities relying on normal brain function. For example, cognition, sensory perception, social behavior and communication and motor skills may all be affected in Fragile X syndrome and autism. These conditions result from a failure of normal brain development, with problems occurring at the microscopic anatomical level, rather than gross compromise of the structure of the brain.

Fragile X Syndrome is a genetically determined severe neurological disorder in which affected individuals are intellectually handicapped to varying degrees and display a variety of psychiatric symptoms. The disorder is primarily caused by the transcriptional silencing of the *FRM1* gene (Verkerk et al. Cell vol 65 1991 pp 905-914), leading to the functional equivalent of a monogenetic disorder (Hagerman West J Med vol 166 1997 pp 129 - 137). Fragile X Syndrome is the largest single cause of inherited intellectual handicap. There are no approved treatments for Fragile X Syndrome. There is therefore a medical need for novel therapeutics that addresses the underlying pathology of this disorder.

Autism is a term used to describe the Autistic spectrum disorders, otherwise known as pervasive developmental disorders, meaning Autistic Disorder, Asperger Syndrome and Pervasive Developmental Disorder Not Otherwise Specified (PDD-NOS). These conditions are recognised diagnostic entities according to the Diagnostic and Statistical Manual of Mental Disorders Revision (DSM-IV) and the International Statistical Classification of Diseases and Related Health Problems (ICD-10) (Tidmarsh and Volkmar Can J Psychiatry vol. 48 2003 pp 517-525). Autistic spectrum disorders affect approximately 1 in 150 people (Autism and Developmental Disabilities Monitoring Network Surveillance Year 2002 Principal Investigators; Centers for Disease Control and Prevention, MMWR Surveill Summ. Vol. 56 2007 pp 12-28).

Fragile X syndrome (FXS) is the most common hereditary form of mental retardation. Mutations in FMR1, which encodes the fragile X mental retardation protein (FMRP), are the cause of fragile X syndrome (FXS), an X-linked mental retardation disorder. Inactivation of the mouse gene Fmr1 confers a number of FXS-like phenotypes including an enhanced susceptibility to epileptogenesis during development. The findings of the paper teach that induction of the group I mGluR-mediated, prolonged epileptiform discharges with biculline in a FXS mouse model was inhibited in hippocampal preparations that were pre treated with an inhibitor of ERK1/2 phosphorylation. As ERK1/2 inhibitor the MEK inhibitor PD98059 was used. (S.-C. Chuang: "Prolonged Epileptiform Discharges Induced by Altered Group I Metabotropic Glutamate Receptor-Mediated Synaptic Responses in Hippocampal Slices of a Fragile X Mouse Model",Journal of Neuroscience, JOURNAL OF NEUROSCIENCE, Vol. 25, no. 35, 31 August 2005, pages 8048-8055, XP55093890, ISSN: 0270-6474, DOI: 10.1523/JNEUROSCI. 1777-05.2005).

At present, there are no medications for the treatment of the core symptomatology of autistic spectrum disorders. Two atypical anti-psychotica, RISPERDAL and ABILIFY, are approved for the treatment of irritability in children and adolescents with autistic disorder in the US. There is no well controlled data available to show that any agent has clinical benefit on all core symptoms of autistic spectrum disorder. There is therefore a medical need for novel therapeutics that addresses the underlying pathology of autistic spectrum disorders.

### SUMMARY OF THE INVENTION

This application relates to use of ERK inhibiting compounds for treating a subject at risk of or suspected of having Fragile X syndrome or autism spectrum disorder associated with abnormalities of ERK. The method includes administering to the subject a therapeutically effective amount of at least one ERK inhibiting compound that prevents abnormalities in neuronal connectivity.

In other aspects, the ERK inhibiting compound is selected from the group consisting of 2-(2-amino-3-methoxyphenyl)-4H-chromen-4-one, (2Z,3Z)-bis{amino[(2-aminophenyl)sulfanyl]methylidene}butanedinitrile, 5-[(4-bromo-2-chlorophenyl)amino]-4-fluoro-N-(2-hydroxyethoxy)-1-methyl-1H-benzimidazole-6-carboxamide, 2-[(2-fluoro-4-iodophenyl)amino]-N-(2-hydroxyethoxy)-1,5-dimethyl-6-oxo-1,6-dihydropyridine-3-carboxamide, 2-(4-chloro-2-fluoro-anilino)-N-(2-hydroxyethoxy)-1,5-dimethyl-6-oxo-pyridine-3-carboxamide, 2-[(2-chloro-4-iodophenyl)amino]-N-(cyclopropylmethoxy)-3,4-difluorobenzamide, (3R,4R)-4-(3,4-Dimethoxybenzyl)-3-(4-hydroxy-3-methoxybenzyl)dihydrofuran-2(3H)-one, (2Z)-3-amino-3-[(4-aminophenyl)sulfanyl]-2-[2-(trifluoromethyl)phenyl]prop-2-enenitrile, hypericin, 3-(3-amino-2H-pyrazolo[3,4-c]pyridazin-5-yl)-2-phenyl-3H-pyrazolo[1,5-a]pyridin-8-ium, N-methyl-3-phenyl-3-[4-(trifluoromethyl)phenoxy]propan-1-amine fluoxetine, and 2-chloro-4-{[2-{[(2R)-1-hydroxy-3-methylbutan-2-yl]amino}-9-(propan-2-yl)-9H-purin-6-yl]amino}benzoic acid.

In still other aspects, the ERK inhibiting compound is an inhibitor of MEK selected from the group consisting of 2-(2-amino-3-methoxyphenyl)-4H-chromen-4-one, (2Z,3Z)-bis{amino[(2-aminophenyl)sulfanyl]methylidene}butanedinitrile, 5-[(4-bromo-2-chlorophenyl)amino]-4-fluoro-N-(2-hydroxyethoxy)-1-methyl-1H-benzimidazole-6-carboxamide, 2-[(2-fluoro-4-iodophenyl)amino]-N-(2-hydroxyethoxy)-1,5-dimethyl-6-oxo-1,6-dihydropyridine-3-carboxamide, 2-(4-chloro-2-fluoro-anilino)-N-(2-hydroxyethoxy)-1,5-dimethyl-6-oxo-pyridine-3-carboxamide, 2-[(2-chloro-4-iodophenyl)amino]-N-(cyclopropylmethoxy)-3,4-difluorobenzamide, (3R,4R)-4-(3,4-Dimethoxybenzyl)-3-(4-hydroxy-3-methoxybenzyl)dihydrofuran-2(3H)-one, and (2Z)-3-amino-3-[(4-aminophenyl)sulfanyl]-2-[2-(trifluoromethyl)phenyl]prop-2-enenitrile.

In a further aspect, the ERK inhibiting compound is an inhibitor of ERK selected from the group consisting of hypericin, 3-(3-amino-2H-pyrazolo[3,4-c]pyridazin-5-yl)-2-phenyl-3H-pyrazolo[1,5-a]pyridin-8-ium, N-methyl-3-phenyl-3-[4-(trifluoromethyl)phenoxy]propan-1-amine fluoxetine, and 2-chloro-4-{[2-{[(2R)-1-hydroxy-3-methylbutan-2-yl]amino}-9-(propan-2-yl)-9H-purin-6-yl]amino}benzoic acid.

In other aspects, the ERK inhibiting compound can be in the form of one or more of its stereoisomers. The ERK inhibiting compound can be administered in combination with at least one or more therapeutic agents that is used for the treatment of autistic spectrum disorders. The therapeutic agent can be selected from the group consisting of an anti-depressant, anti-psychotic, stimulant, and other medications. The one or more additional therapeutic agents can be selected from the group consisting of risperidone, aripiprazole, citalopram, escitalopram, sertraline, methylphenidate, atomoxetine, memantine and minocycline.

In still other aspects, the ERK inhibiting compound can be provided in a pharmaceutically acceptable form selected from the group consisting of tablets, troches, lozenges, aqueous and oily suspensions, dispersible powders and granules, emulsions, hard and soft capsules, syrups and elixirs. The ERK inhibiting compound can be formulated for separate, simultaneous, sequential or extended release into a subject.

In a further aspect, the autistic spectrum disorder is selected from autistic disorder, Asperger Syndrome or Pervasive Developmental Disorder Not Otherwise Specified or a subset of these patients in which ERK function is abnormal.

This application also relates to the use of ERK inhibiting compounds singly or in combination that prevent abnormalities in neuronal connectivity; or a pharmaceutically acceptable salt thereof; in the manufacture of a medicament for the treatment of Fragile X syndrome or autism spectrum disorders associated with abnormalities of ERK.

In other aspects, the ERK inhibiting compound is selected from the group consisting of 2-(2-amino-3-methoxyphenyl)-4H-chromen-4-one, (2Z,3Z)-bis{amino[(2-aminophenyl)sulfanyl]methylidene}butanedinitrile, 5-[(4-bromo-2-chlorophenyl)amino]-4-fluoro-N-(2-hydroxyethoxy)-1-methyl-1 H-benzimidazole-6-carboxamide, 2-[(2-fluoro-4-iodophenyl)amino]-N-(2-hydroxyethoxy)-1,5-dimethyl-6-oxo-1,6-dihydropyridine-3-carboxamide, 2-(4-chloro-2-fluoro-anilino)-N-(2-hydroxyethoxy)-1,5-dimethyl-6-oxo-pyridine-3-carboxamide, 2-[(2-chloro-4-iodophenyl)amino]-N-(cyclopropylmethoxy)-3,4-difluorobenzamide, (3R,4R)-4-(3,4-Dimethoxybenzyl)-3-(4-hydroxy-3-methoxybenzyl)dihydrofuran-2(3H)-one, (2Z)-3-amino-3-[(4-aminophenyl)sulfanyl]-2-[2-(trifluoromethyl)phenyl]prop-2-enenitrile, hypericin, 3-(3-amino-2H-pyrazolo[3,4-c]pyridazin-5-yl)-2-phenyl-3H-pyrazolo[1,5-a]pyridin-8-ium, N-methyl-3-phenyl-3-[4-(trifluoromethyl)phenoxy]propan-1-amine fluoxetine, and 2-chloro-4-{[2-{[(2R)-1-hydroxy-3-methylbutan-2-yl]amino}-9-(propan-2-yl)-9H-purin-6-yl]amino}benzoic acid.

In still other aspects, the ERK inhibiting compound is an inhibitor of MEK selected from the group consisting of 2-(2-amino-3-methoxyphenyl)-4H-chromen-4-one, (2Z,3Z)-bis{amino[(2-aminophenyl)sulfanyl]methylidene}butanedinitrile, 5-[(4-bromo-2-chlorophenyl)amino]-4-fluoro-N-(2-hydroxyethoxy)-1-methyl-1H-benzimidazole-6-carboxamide, 2-[(2-fluoro-4-iodophenyl)amino]-N-(2-hydroxyethoxy)-1,5-dimethyl-6-oxo-1,6-dihydropyridine-3-carboxamide, 2-(4-chloro-2-fluoro-anilino)-N-(2-hydroxyethoxy)-1,5-dimethyl-6-oxo-pyridine-3-carboxamide, 2-[(2-chloro-4-iodophenyl)amino]-N-(cyclopropylmethoxy)-3,4-difluorobenzamide, (3R,4R)-4-(3,4-Dimethoxybenzyl)-3-(4-hydroxy-3-methoxybenzyl)dihydrofuran-2(3H)-one, and (2Z)-3-amino-3-[(4-aminophenyl)sulfanyl]-2-[2-(trifluoromethyl)phenyl]prop-2-enenitrile.

In a further aspect, the ERK inhibiting compound is an inhibitor of ERK selected from the group consisting of hypericin, 3-(3-amino-2H-pyrazolo[3,4-c]pyridazin-5-yl)-2-phenyl-3H-pyrazolo[1,5-a]pyridin-8-ium, N-methyl-3-phenyl-3-[4-(trifluoromethyl)phenoxy]propan-1-amine fluoxetine, and 2-chloro-4-{[2-{[(2R)-1-hydroxy-3-methylbutan-2-yl]amino} -9-(propan-2-yl)-9H-purin-6-yl]amino}benzoic acid.

In other aspects, the ERK inhibiting compound can be in the form of one or more of its stereoisomers. The medicament can also one or more therapeutic agents that is used for the treatment of autistic spectrum disorders in combination with the ERK inhibiting compound. The therapeutic agent can be selected from the group consisting of an anti-depressant, anti-psychotic, stimulant, and other medications. The one or more additional therapeutic agents can be selected from the group consisting of risperidone, aripiprazole, citalopram, escitalopram, sertraline, methylphenidate, atomoxetine, memantine and minocycline.

In still other aspects, the ERK inhibiting compound can be provided in a pharmaceutically acceptable form selected from the group consisting of tablets, troches, lozenges, aqueous and oily suspensions, dispersible powders and granules, emulsions, hard and soft capsules, syrups and elixirs. The ERK inhibiting compound can be formulated for separate, simultaneous, sequential or extended release into a subject.

In a further aspect, the autistic spectrum disorder is selected from autistic disorder, Asperger Syndrome or Pervasive Developmental Disorder Not Otherwise Specified or a subset of these patients in which ERK function is abnormal.

### DESCRIPTION OF THE DRAWINGS

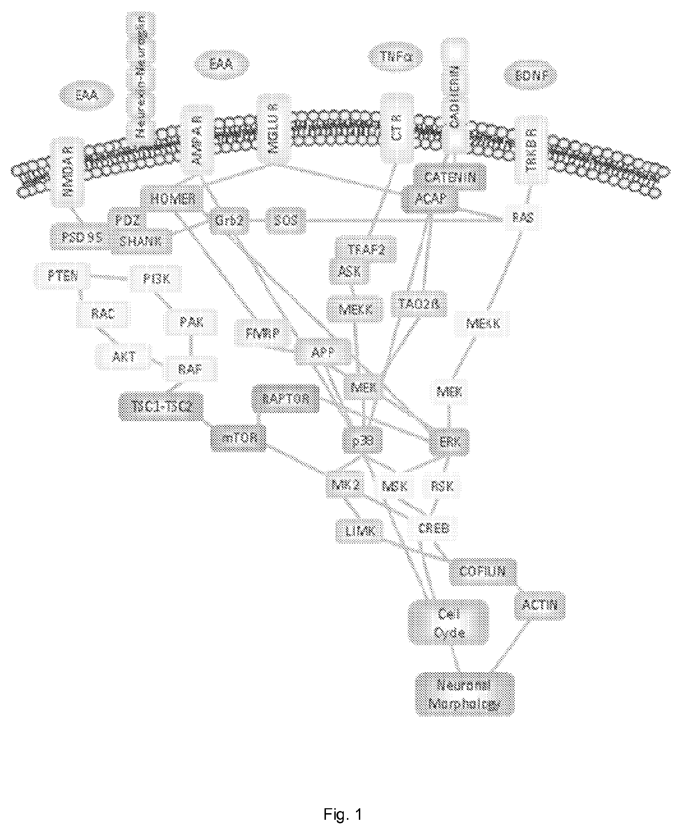

Fig. 1 illustrates intracellular MAPK pathways relevant to neuronal plasticity and connectivity, dendritic morphology, and brain function

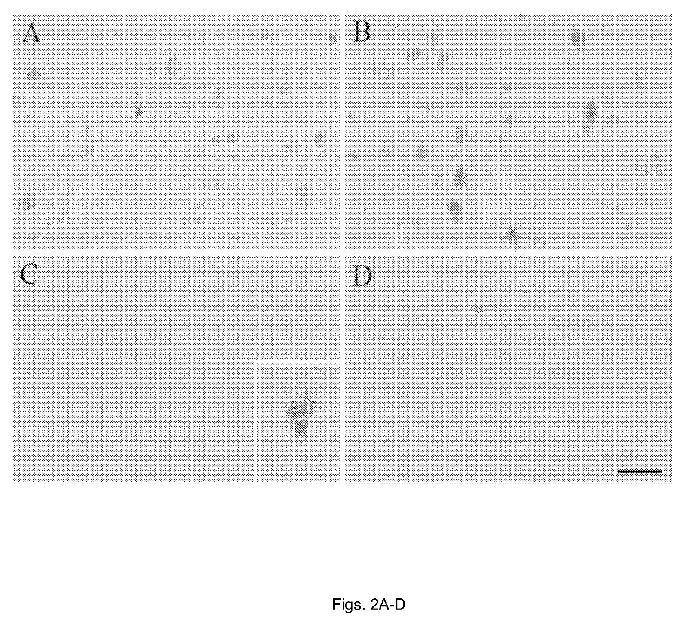

Fig. 2 illustrates images showing pERK localization in sections of hippocampus. The highest levels of pERK in neuronal nuclei and cytoplasm were found in cases of FXS. Representative areas of the CA1/CA2 region are shown of the 9 year (A) and 21 year (B) old cases of FXS. All control cases contained nominal levels of pERK in the hippocampus (20 year old, C) Inset in C shows pERK localization to granulovascular degeneration found in a very small number of pyramidal neurons in a 67 year old control case, changes which are consistent with normal aging. A 71 year old case of FXTAS shows relatively low levels of neuronal pERK (D) compared to the cases of FXS. Scale bar = 50 µm.

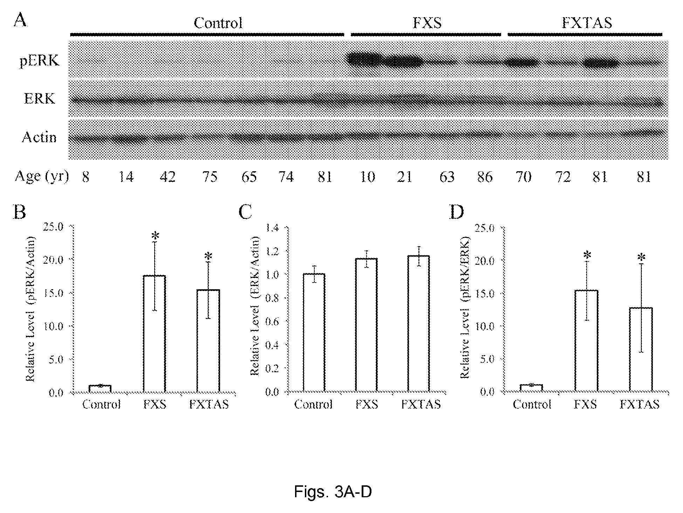

Fig. 3 illustrates an immunoblot (A) and charts showing quantification analysis (B and C) of ERK and phospho-ERK (p-ERK) in samples from 4 FXS cases, 4 FXTAS cases, and 7 age-matched controls. The charts indicate that, although relative ERK level (ERK/Actin) did not change significantly (B), the relative p-ERK level (B), the relative ERK level (C) and the relative ratio of p-ERK/ERK (D) increased significantly in both FXS and FXTAS brain samples. All samples were also immunoblotted with antibodies to detect actin. (*p < 0.05, student-t-test).

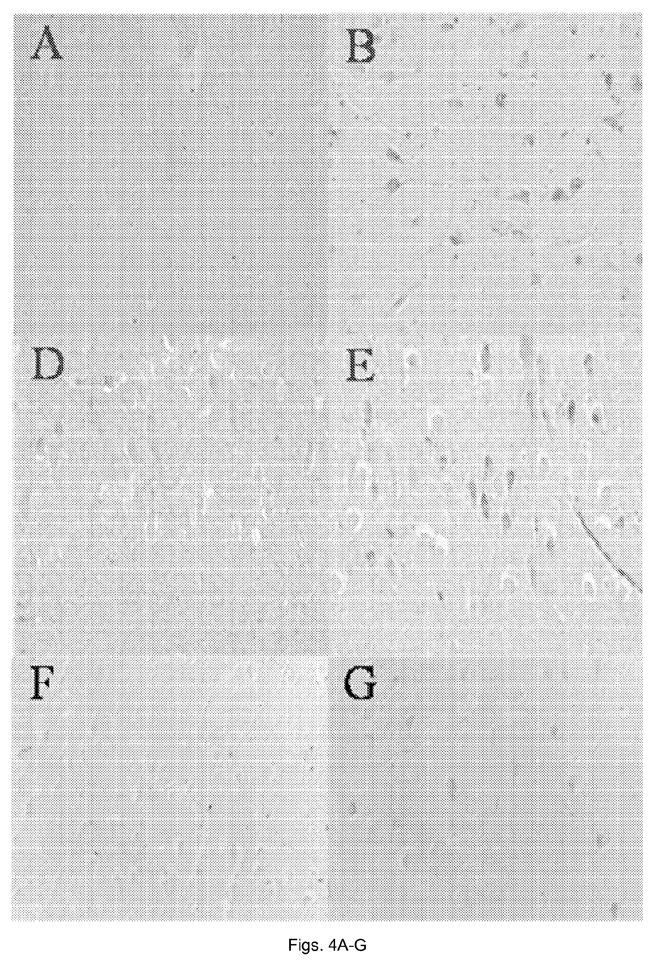

Fig. 4 illustrates images showing expression of phosphor-ERK and related proteins in brain in autistic spectrum disorders and in the neurologically normal. Photomicrographs show immunohistochemical staining of phosphorylated proteins labelled using phospho antibodies (Cell Signaling Technologies) and diaminobenzidine as chromogen. Tissue is cortex. Autistic patients were ages 9 and 11 with a diagnosis of autism. First row shows tissue from a normal subject (A) and a patient with autism (B) stained for pERK. Second row shows tissue from a normal subject (C) and a patient with autism (D) stained for pMEK. Third row shows tissue from a normal subject (E) and a patient with autism (F) stained for pMSK2.

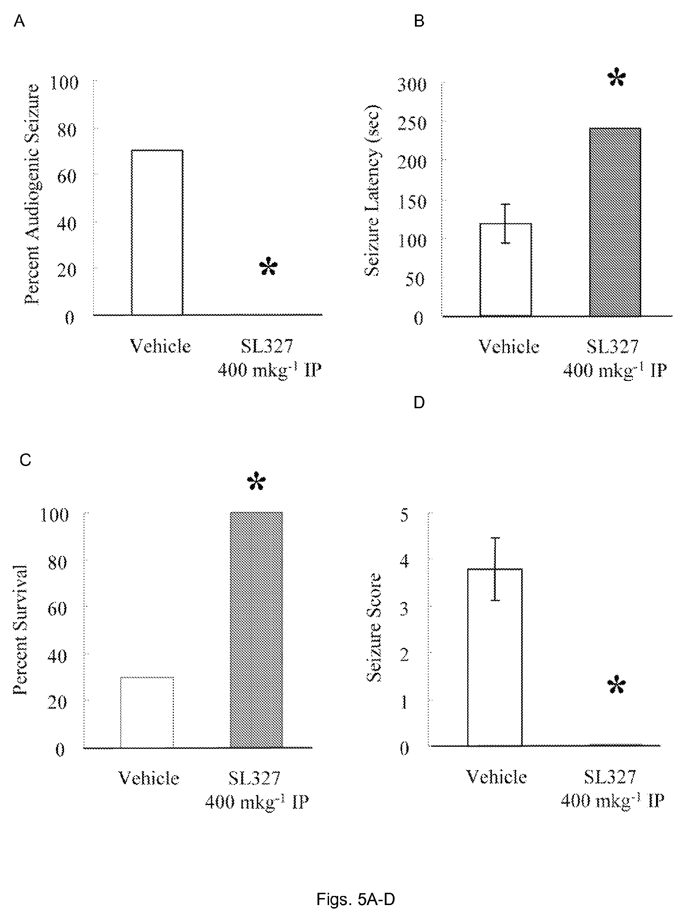

Fig. 5 illustrates the charts showing the effect of SL327 (400 mg.kg.⁻¹ IP) on audiogenic seizures in the *fmr1* knockout transgenic mouse model of Fragile X syndrome, compared to Tween80 vehicle treated control animals. Data are from n = 10 animals per group. Treatment with SL327 completely inhibits all seizure activity in a mice treated, while approximately 70% of untreated animals displayed audiogenic-induced seizures. The latency (seconds) to seizure onset is also significantly reduced in the treated group. Since the treated mice did not exhibit any seizure activity, their latency score is reported as 240 sec, the maximum total time of audiogenic stimulation. The survival rate following testing is also increased from 25% to 100% following treatment with SL327.

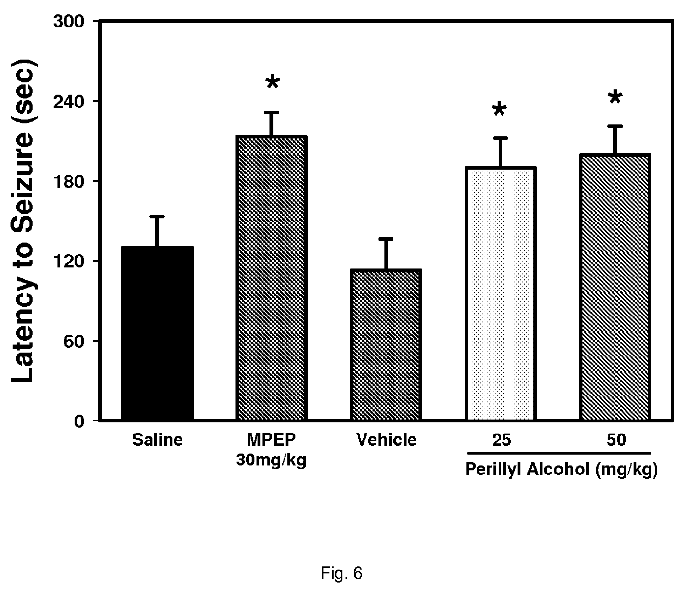

Fig. 6 illustrates a chart showing the effects of MPEP and perillyl alcohol on latency to seize. Data are presented as mean ± SEM. Asterisks (*p<0.05) indicate a significant difference compared to respective vehicle.

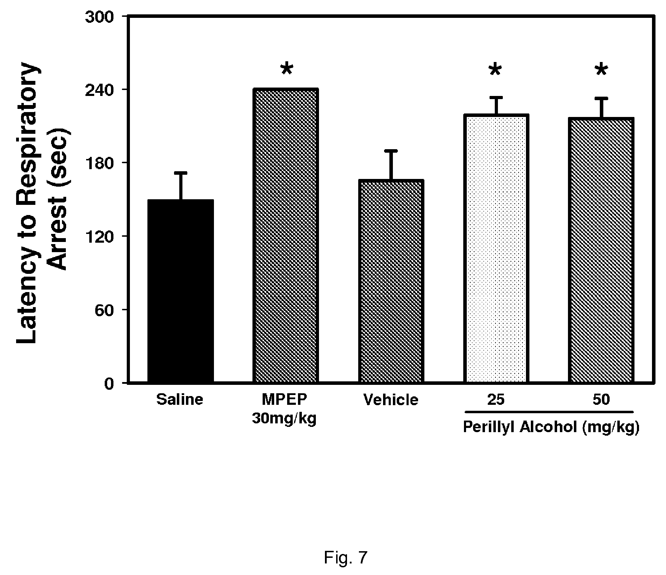

Fig. 7 illustrates a chart showing the effects of MPEP and perillyl alcohol on latency to respiratory arrest. Data are presented as mean ± SEM. Asterisks (*p<0.05) indicate a significant difference compared to respective vehicle.

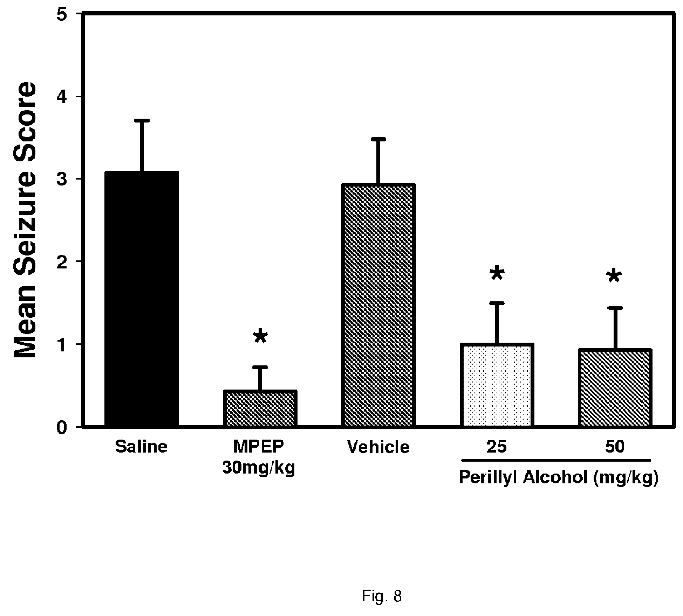

Fig. 8 illustrates a chart showing the effects of MPEP and perillyl alcohol on mean seizure score. Data are presented as mean ± SEM. Asterisks (*p<0.05) indicate a significant difference compared to respective vehicle.

### DESCRIPTION OF THE INVENTION

Unless specifically addressed herein, all terms used have the same meaning as would be understood by those of skilled in the art of the present invention. The following definitions will provide clarity with respect to the terms used in the specification and claims to describe the present invention.

The articles "a" and "an" are used herein to refer to one or to more than one (*i.e*., to at least one) of the grammatical object of the article. By way of example, "an element" means one element or more than one element.

The terms "comprise," "comprising," "include," "including," "have," and "having" are used in the inclusive, open sense, meaning that additional elements may be included. The terms "such as", "*e.g*.", as used herein are non-limiting and are for illustrative purposes only. "Including" and "including but not limited to" are used interchangeably.

The term "or" as used herein should be understood to mean "and/or", unless the context clearly indicates otherwise.

It will be noted that the structure of some of the compounds of the application include asymmetric (chiral) carbon atoms. It is to be understood accordingly that the isomers arising from such asymmetry are included within the scope of the invention, unless indicated otherwise. Such isomers can be obtained in substantially pure form by classical separation techniques and by stereochemically controlled synthesis. The compounds of this application may exist in stereoisomeric form, therefore can be produced as individual stereoisomers or as mixtures.

"Isomerism" means compounds that have identical molecular formulae but that differ in the nature or the sequence of bonding of their atoms or in the arrangement of their atoms in space. Isomers that differ in the arrangement of their atoms in space are termed "stereoisomers". Stereoisomers that are not mirror images of one another are termed "diastereoisomers", and stereoisomers that are non-superimposable mirror images are termed "enantiomers", or sometimes optical isomers. A carbon atom bonded to four nonidentical substituents is termed a "chiral center".

"Chiral isomer" means a compound with at least one chiral center. It has two enantiomeric forms of opposite chirality and may exist either as an individual enantiomer or as a mixture of enantiomers. A mixture containing equal amounts of individual enantiomeric forms of opposite chirality is termed a "racemic mixture". A compound that has more than one chiral center has 2n-1 enantiomeric pairs, where n is the number of chiral centers. Compounds with more than one chiral center may exist as either an individual diastereomer or as a mixture of diastereomers, termed a "diastereomeric mixture". When one chiral center is present, a stereoisomer may be characterized by the absolute configuration (R or S) of that chiral center. Absolute configuration refers to the arrangement in space of the substituents attached to the chiral center. The substituents attached to the chiral center under consideration are ranked in accordance with the Sequence Rule of Cahn, Ingold and Prelog. (Cahn et al, Angew. Chem. Inter. Edit. 1966, 5, 385; errata 511; Cahn et al., Angew. Chem. 1966, 78, 413; Cahn and Ingold, J Chem. Soc. 1951 (London), 612; Cahn et al., Experientia 1956, 12, 81; Cahn, J., Chem. Educ. 1964, 41, 116).

"Geometric Isomers" means the diastereomers that owe their existence to hindered rotation about double bonds. These configurations are differentiated in their names by the prefixes *cis* and *trans,* or Z and E, which indicate that the groups are on the same or opposite side of the double bond in the molecule according to the Cahn-Ingold-Prelog rules.

Further, the structures and other compounds discussed in this application include all atropic isomers thereof. "Atropic isomers" are a type of stereoisomer in which the atoms of two isomers are arranged differently in space. Atropic isomers owe their existence to a restricted rotation caused by hindrance of rotation of large groups about a central bond. Such atropic isomers typically exist as a mixture, however as a result of recent advances in chromatography techniques, it has been possible to separate mixtures of two atropic isomers in select cases.

As used herein, the term "Fragile X syndrome" refers to the disorder caused by abnormalities in function of the *FMR1* gene, affecting around 1 in 4000 of the population.

As used herein, the term "autistic spectrum disorder" refers to psychiatric or neurological disorders attributable to diseases of the nervous system. Examples of such disorders are autistic disorder, Asperger Syndrome and Pervasive Developmental Disorder Not Otherwise Specified (PDD-NOS). It is estimated that 1 in 150 of the general population suffer from autistic spectrum disorders, representing 1.8 million patients in the US and over 3 million patients in the EU that could benefit from the present invention.

As used herein, the terms "patient" and "subject" refer to any animal, including, but not limited to, humans and non-human animals (*e*.*g*., rodents, arthropods, insects, fish (*e*.*g*., zebrafish), non-human primates, ovines, bovines, ruminants, lagomorphs, porcines, caprines, equines, canines, felines, aves, etc.), which is to be the recipient of a particular treatment. Typically, the terms "patient" and "subject" are used interchangeably herein in reference to a human subject.

As used herein, the term "modulate" refers to a change in the biological activity of a biologically active molecule. Modulation can be an increase or a decrease in activity, a change in binding characteristics, or any other change in the; biological, functional, or immunological properties of biologically active molecules.

As used herein, the term "*in vitro*" refers to an artificial environment and to processes or reactions that occur within an artificial environment. In vitro environments consist of, but are not limited to, test tubes and cell culture.

As used herein, the term "*in vivo*" refers to the natural environment (*e.g*., an animal or a cell) and to processes or reaction that occur within a natural environment.

As used herein, the term "test compound" refers to any chemical entity, pharmaceutical, drug, and the like that are used to treat or prevent a disease, illness) sickness or disorder of bodily function. Test compounds comprise both known and potential therapeutic compounds. A test compound can be determined to be therapeutic by screening using the screening methods of the present invention. A "known therapeutic compound" refers to a therapeutic compound that has been shown (*e*.*g*., through animal trials or prior experience with administration to humans) to be effective in such treatment or prevention.

The term "treating" is art-recognized and includes inhibiting a disease, disorder or condition in a subject, e.g., impeding its progress; and relieving the disease, disorder or condition, *e*.*g*., causing regression of the disease, disorder and/or condition. Treating the disease or condition includes ameliorating at least one symptom of the particular disease or condition, even if the underlying pathophysiology is not affected.

The term "preventing" is art-recognized and includes stopping a disease, disorder or condition from occurring in a subject, which may be predisposed to the disease, disorder and/or condition but has not yet been diagnosed as having it. Preventing a condition related to a disease includes stopping the condition from occurring after the disease has been diagnosed but before the condition has been diagnosed.

As used herein, the term "reduction" of a symptom or symptoms (and grammatical equivalents of this phrase) refers to decreasing of the severity or frequency of the symptom(s), or elimination of the symptom(s).

As used herein, the terms "administering" or "administration of" a drug to a subject (and grammatical equivalents of this phrase) includes both direct administration, including self-administration, and indirect administration, including the act of prescribing a drug. For example, as used herein, a physician who instructs a patient to self-administer a drug and/or provides a patient with a prescription for a drug is administering the drug to the patient.

As used herein, a "manifestation" of a disease refers to a symptom, sign, anatomical state, physiological state, or report characteristic of a subject with the disease.

As used herein, a "therapeutically effective amount" of a drug or agent is an amount of a drug or agent that, when administered to a subject with a disease or condition, will have the intended therapeutic effect, *e*.*g*., alleviation, amelioration, palliation or elimination of one or more manifestations of the disease or condition in the subject. The full therapeutic effect does not necessarily occur by administration of one dose and may occur only after administration of a series of doses. Thus, a therapeutically effective amount may be administered in one or more administrations.

As used herein, a "prophylactically effective amount" of a drug is an amount of a drug that, when administered to a subject, will have the intended prophylactic effect, *e*.*g*., preventing or delaying the onset (or reoccurrence) of disease or symptoms, or reducing the likelihood of the onset (or reoccurrence) of disease or symptoms. The full prophylactic effect does not necessarily occur by administration of one dose and may occur only after administration of a series of doses. Thus, a prophylactically effective amount may be administered in one or more administrations.

The phrases "parenteral administration" and "administered parenterally" are art-recognized terms, and include modes of administration other than enteral and topical administration, such as injections, and include, without limitation, intravenous, intramuscular, intrapleural, intravascular, intrapericardial, intraarterial, intrathecal, intracapsular, intraorbital, intracardiac, intradermal, intraperitoneal, transtracheal, subcutaneous, subcuticular, intra-articular, subcapsular, subarachnoid, intraspinal and intrastemal injection and infusion.

The phrases "systemic administration," "administered systemically," "peripheral administration" and "administered peripherally" as used herein mean the administration of a compound, drug or other material other than directly into the central nervous system, such that it enters the animal's system and, thus, is subject to metabolism and other like processes, for example, subcutaneous administration.

A "pharmaceutical composition" is a formulation containing the disclosed compounds in a form suitable for administration to a subject. In a preferred embodiment, the pharmaceutical composition is in bulk or in unit dosage form. The unit dosage form is any of a variety of forms, including, for example, a capsule, an IV bag, a tablet, a single pump on an aerosol inhaler, or a vial. The quantity of active ingredient (*e*.*g*., a formulation of the disclosed compound or salts thereof) in a unit dose of composition is an effective amount and is varied according to the particular treatment involved. One skilled in the art will appreciate that it is sometimes necessary to make routine variations to the dosage depending on the age and condition of the patient. The dosage will also depend on the route of administration. A variety of routes are contemplated, including oral, pulmonary, rectal, parenteral, transdermal, subcutaneous, intravenous, intramuscular, intraperitoneal, intranasal, and the like. Dosage forms for the topical or *transdermal* administration of a compound of this invention include powders, sprays, ointments, pastes, creams, lotions, gels, solutions, patches and inhalants. In a preferred embodiment, the active compound is mixed under sterile conditions with a pharmaceutically acceptable carrier, and with any preservatives, buffers, or propellants that are required.

The term "flash dose" refers to compound formulations that are rapidly dispersing dosage forms.

The term "immediate release" is defined as a release of compound from a dosage form in a relatively brief period of time, generally up to about 60 minutes. The term "modified release" is defined to include delayed release, extended release, and pulsed release. The term "pulsed release" is defined as a series of releases of drug from a dosage form. The term "sustained release" or "extended release" is defined as continuous release of a compound from a dosage form over a prolonged period.

The phrase "pharmaceutically acceptable" is art-recognized. In certain embodiments, the term includes compositions, polymers and other materials and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically acceptable carrier" is art-recognized, and includes, for example, pharmaceutically acceptable materials, compositions or vehicles, such as a liquid or solid filler, diluent, excipient, solvent or encapsulating material, involved in carrying or *trans*porting any subject composition from one organ, or portion of the body, to another organ, or portion of the body. Each carrier must be "acceptable" in the sense of being compatible with the other ingredients of a subject composition and not injurious to the patient. In certain embodiments, a pharmaceutically acceptable carrier is non-pyrogenic. Some examples of materials which may serve as pharmaceutically acceptable carriers include: (1) sugars, such as lactose, glucose and sucrose; (2) starches, such as corn starch and potato starch; (3) cellulose, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, sunflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

The compounds of the application are capable of further forming salts. All of these forms are also contemplated within the scope of the claimed invention.

"Pharmaceutically acceptable salt" of a compound means a salt that is pharmaceutically acceptable and that possesses the desired pharmacological activity of the parent compound. For example, the salt can be an acid addition salt. One embodiment of an acid addition salt is a hydrochloride salt. The pharmaceutically acceptable salts can be synthesized from a parent compound that contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, non-aqueous media like ether, ethyl acetate, ethanol, isopropanol, or acetonitrile are preferred. Lists of salts are found in Remington's Pharmaceutical Sciences, 18th ed. (Mack Publishing Company, 1990). For example, salts can include, but are not limited to, the hydrochloride and acetate salts of the aliphatic amine-containing, hydroxyl amine-containing, and imine-containing compounds of the present invention.

It should be understood that all references to pharmaceutically acceptable salts include solvent addition forms (solvates) or crystal forms (polymorphs) as defined herein, of the same salt.

Administration of an agent "in combination with" or "in conjunction with" includes parallel administration (administration of both the agents to the patient over a period-of time, such as administration on alternate days for one month), co-administration (in which the agents are administered at approximately the same time, e.g., within about a few minutes to a few hours of one another), and co-formulation (in which the agents are combined or compounded into a single dosage form suitable for oral or parenteral administration).

This application relates to compositions and methods of treating a subject at risk of or suspected of having Fragile X syndrome or autism spectrum disorder associated with abnormalities of ERK.

There is overlap of the symptomatology of autism and Fragile X Syndrome that is paralleled by overlap in the biological features of these disorders. This overlap may be seen at the level of microscopic investigation of the structure of neurons on both conditions. In Fragile X Syndrome, the fine structure of neuronal processes is altered. That is, dendritic spine density is increased in Fragile X Syndrome. The fine structure of neuronal processes is also altered in some subjects with autism. Once cases of non-specific disability are excluded, dendrite spine density is increased in some subjects with autism. This increase becomes more pronounced as the severity of symptoms worsens.

The density of dendritic spines reflects the number of synaptic connections between neurons, and is subject to change. It was found that the impairments in brain function seen in Fragile X syndrome and autism result from impairments in control of the plasticity of connections between neurons as mediated by changes in spine density. Neurotrophic factors, such as BDNF, are increased in autism, therefore potentially increasing spine density. In Fragile X Syndrome, a loss of FRMP results in a failure of LTD and therefore a failure of the induction of synaptic pruning or appropriate reduction in spine density. It is therefore suggested that autism and Fragile X Syndrome impairments of brain function arise from either an inappropriate induction of increase in spine density, or a failure to decrease spine density.

The induction of spine density relies on activation of extracellular signal-regulated kinase (ERK). Induction of ERK is required for initiation of the activation of transcription factors and synthesis of proteins in maintenance of LTP and generation of dendritic spines. Fig. 1 shows the intracellular signalling pathway underlying activity of ERK in relation to this function and neuronal plasticity and connectivity.

The function of ERK has been studied in Fragile X syndrome. A number of studies using neuronal and peripheral tissue from a transgenic mouse model of Fragile X syndrome (the *fmr1* KO mouse) and peripheral tissue from FXS patients have investigated ERK phosphorylation. Hou et al. (Hou, Antion, Hu et al. Neuron. Vol. 51 2006 pp 441-454) showed that phosphorylation of ERK seen after metabotropic glutamate (mGluR) receptor stimulation in control animals was absent. This absence of stimulation induced ERK phosphorylation was replicated by Kim et al. (Kim SH, Markham JA, Weiler IJ, Greenough WT. Proc Natl Acad Sci USA. Vol. 105 2008 pp 4429-4434) who reported an absence of stimulation induced ERK phosphorylation in cortical synaptoneurosomes from *fmr1* KO mice. Indeed, in this study, mGluR mediated stimulation induced a persistent dephosphorylation of ERK, mediated by inappropriate activation of phosphatases. This deficient induction of ERK phosphorylation in *fmr1* KO mouse brain was replicated and extended by Weng et al. (Weng N, Weiler IJ, Sumis A, Berry-Kravis E, Greenough WT. Am J Med Genet B Neuropsychiatr Genet. Vol. 147B 2008 pp 1253-1257) who reported stimulated ERK phosphorylation was impaired in peripheral tissue from *fmr1* KO mice and FXS patients. Overall, this data would imply that levels of phosphorylated ERK are depleted in Fragile X Syndrome.

The prediction above that ERK phosphorylation should be reduced in Fragile X syndrome had not been tested in brain tissue from patients with this condition. Accordingly, post mortem human brain tissue from FXS and FXTAS patients was examined with respect to phosphorylated ERK levels. Surprisingly, phosphorylated ERK levels were increased when visualized using immunohistochemcial staining (Fig. 2). Western blot analysis was used to confirm this finding (Fig. 3). Since Fragile X syndrome and autism appear to overlap in terms of presenting symptoms and in some cases with respect to the dendritic structure of neurons, autism brain was also investigated (Fig. 4). ERK levels are increased in autism as well, as would be predicted by a model of these disorders in which inappropriate induction of increases in spine density had occurred.

The data shown in the examples suggest that ERK activity is abnormally increased in the brain in Fragile X Syndrome and at least a subset of patients with autism. Further, this abnormal increase in ERK activity is a final common pathway within neurons wherein disease inducing factors, such as a loss of FMRP, genetic polymorphisms in cadherins and environmental factors increasing cytokine and neurotrophin levels are translated into abnormal changes in spine density. Altered spine density in turn results in altered neuronal plasticity and therefore disturbances in brain function.

It was found that inhibition of excess, enhanced, or abnormally high ERK activity reverses symptoms of Fragile X syndrome and some cases of autism. By way of example, the effect of inhibition was investigated in *fmr1* KO mice. Like pediatric cases of Fragile X syndrome, juvenile *fmr1* KO mice are predisposed to epilepsy. A predisposition to epilepsy is also common in autism. Therefore audiogenic seizure activity was investigated in *fmr1* KO mice in the presence of treatment with either control vehicle solution or the compound SL 327. SL 327 is a potent and selective inhibitor of MEK. Since MEK is an upstream activator of ERK, inhibition with SL327 will decrease ERK activity. SL327 remarkably antagonized the development of audiogenic seizures in *fmr1* KO mice, e.g., seizure activity was completely prevented by administration of this compound.

Without wishing to be bound by theory, it is believed that inhibition of ERK, either directly or via modulation of its up- or down- stream control elements, will prevent abnormal regulation of neuronal plasticity caused by genetic abnormalities or the interaction between genetic abnormality and environmental stimuli in Fragile X Syndrome and autism. Accordingly, modulators of neuronal ERK function can be used to restore correct function in autism and Fragile X Syndrome and so reduce symptoms of these disorders. A treatment for these types of disorders can be one that deactivates the increase in ERK function seen in Fragile X syndrome or autism.

An aspect of the application therefore relates to compositions and methods of treating a subject at risk of or suspected of having Fragile X syndrome or autism spectrum disorders associated with abnormalities of ERK by administering to the subject a therapeutically effective amount of an ERK inhibiting compounds, that prevents abnormalities in neuronal connectivity. The ERK inhibiting compound can include any compound that act on signaling pathways involving ERK and that prevent abnormalities in neuronal connectivity.

In some embodiments of the application, the ERK inhibiting compound can include indirect inhibitors of MAPK acting through MEK, such as inhibitors of MEK. Examples of inhibitors of MEK can include 2-(2-amino-3-methoxyphenyl)-4H-chromen-4-one (PD98059) (Romerio and Zella FASEB J. Vol. 16 2002 pp.1680-1682), (2Z,3Z)-bis{amino[(2-aminophenyl)sulfanyl]methylidene}butanedinitrile (U0126) (Duncia et al. Bioorg Med Chem Lett. Vol. 8 1998 pp. 2839-2844), 5-[(4-bromo-2-chlorophenyl)amino]-4-fluoro-N-(2-hydroxyethoxy)-1-methyl-1H-benzimidazole-6-carboxamide (AZD6244 (ARRY-142886)) (Yeh et al. Clin Cancer Res vol. 13 2007 pp 1576-1583), 2-[(2-fluoro-4-iodophenyl)amino]-N-(2-hydroxyethoxy)-1,5-dimethyl-6-oxo-1,6-dihydropyridine-3-carboxamide (or 2-(4-chloro-2-fluoro-anilino)-N-(2-hydroxyethoxy)-1,5-dimethyl-6-oxo-pyridine-3-carboxamide) AZD8330 (ARRY-424704) (Chung et al Clin Cancer Res. Vol. 15 2009 pp 3050-3057), 2-[(2-chloro-4-iodophenyl)amino]-N-(cyclopropylmethoxy)-3,4-difluorobenzamide (CI-1040) (LoRusso et al. (2005) J Clin Oncol vol. 23 2005 pp 5281-5293), (3R,4R)-4-(3,4-Dimethoxybenzyl)-3-(4-hydroxy-3-methoxybenzyl)dihydrofuran-2(3H)-one (Arctigenin) (Jang et al. J.Neurosci.Res. vol 68 2002 pp 233-240), (2Z)-3-amino-3-[(4-aminophenyl)sulfanyl]-2-[2-(trifluoromethyl)phenyl]prop-2-enenitrile (SL327) (Wang et al. J.Pharmacol.Exp.Ther. vol. 304 2003 pp 172-178), and perillyl alcohol or [(4S)-4-(prop-1-en-2-yl)cyclohex-1-en-1-yl]methanol, which is an inhibitor of MEK (Clark et al Clin Canc Res 9 2003 pp 4494-4505).

Additional examples of MEK inhibitors can include U0125(1,4-Diamino-2,3-dicyano-1,4-bis(phenylthio)butadiene), PD 0325901, AS703026, ARRY-438162, GDC-0973, GSK1120212, RO4987655, RDEA119, TAK-733, E6201, CI-1040, PD 318088, PD 0316684, PD 0188563, PD 169842, PD 0335676, PD 0184264, PD184352, ARRY-509, AR-00241389, GC63, G8935, Isothiazole, LL,Z-1640, Hypothemycin, L-783,277, 10-Z-Hymenialdisine, RO-09-2210, 87-250940, XL-518, AR119, AS-701173, AS-701255, 360770-54-3, NAMI-A , 6-(4-Bromo-2-chloro-phenylamino)-7-fluoro-3 (tetrahydro-pyran-2-ylmefhyl)-3H-benzoimidazole-5 carboxylic acid (2-hydroxy-ethoxy)-amide, 1 -[6-(4-Bromo-2-chloro-phenylamino)-7-fluoro-3 methyl-3H-benzoimidazol-5-yl]-2-hydroxy-ethanone, 6-(4-Bromo-2-chloro-phenylamino)-7-fluoro-3 methyl-3H-benzoimidazole-5-carboxylic acid (2-hydroxy-1,1 -dimethyl-ethoxy)-amide, 6-(4-Bromo-2-chloro-phenylamino)-7-fluoro-3 (tetrahydro-furan-2-ylmethyl)-3H-benzoimidazole-5-carboxylic acid (2-hydroxy-ethoxy)-amide, 6-(4-Bromo-2-fluoro-phenylamino)-7-fluoro-3 methyl-3H-benzoimidazole-5-carboxylic acid (2-hydroxy-ethoxy)-amide and 6-(2,4-Dichloro-phenylamino)-7-fluoro-3-methyl 3H-benzoimidazole-5-carboxylic acid (2-hydroxyethoxy)-amide, 6-(4-Bromo-2-chloro-phenylamino)-7-fluoro-3 methyl-3H-benzoimidazole-5-carboxylic acid (2-hydroxy-ethoxy)-amide, referred to hereinafter as MEK inhibitor 1; 2-[(2-fluoro-4-iodophenyl)amino]-N-(2-hydroxyethoxy)-1,5-dimethyl-6-oxo-1,6-dihydropyridine-3-carboxamide; referred to hereinafter as MEK inhibitor 2; 4-(4-bromo-2-fluorophenylamino)-N-(2-hydroxyethoxy)-1,5-dimethyl-6-oxo-1,6-dihydropyridazine-3 carboxamide 4-(4-Bromo-2-fluorophenylamino)-N-(2-hydroxyethoxy)-1,5-dimethyl-6-oxo-1,6-dihydropyridazine-3-carboxamide, 6-(4-Bromo-2-chloro-phenylamino)-7-fluoro-3-methyl-3H-benzoimidazole-5-carboxylic acid (2-hydroxy-ethoxy)-amide, 2-(2-fluoro-4-iodophenylamino)-N-(2-hydroxyethoxy)-1,5-dimethyl-6-oxo-1,6-dihydropyridine-3-carboxamide, 2-{[3-({4-[(5-{2-[(3 fluorophenyl)amino]-2-oxoethyl}-1H-pyrazol-3-yl)amino]quinazolin-7-yl}oxy)propyl] (ethyl)amino}ethyl dihydrogen phosphate and pharmaceutically acceptable salts thereof.

Still other examples of inhibitors of MEK are described in U.S. Patent Nos. 7,517,994, 7,732,616, 7,893,065, 6,147,107, 6,319,955, 7,528,166, 6,063,383, 6,703,420, 6,972,298, 7,169,816, 6,696,440, 7,235,537, 7,425,637, 5,525,625, 6,150,401, 6,346,282, 6,949,558, 6,573,044, and 6,512,010,.

In other embodiments, the ERK inhibiting compound can include inhibitors of ERK. Examples of inhibitors of ERK can include Hypericin (Romerio and Zella FASEB J. Vol. 16 2002 pp.1680-1682), 3-(3-amino-2H-pyrazolo[3,4-c]pyridazin-5-yl)-2-phenyl-3H-pyrazolo[1,5-a]pyridin-8-ium (FR 180204) ((Ohori et al. Biochem Biophys Res Commun. Vol. 336 2005 pp. 357-363), N-methyl-3-phenyl-3-[4-(trifluoromethyl)phenoxy]propan-1-amine fluoxetine (Valjent et al Eur J Neurosci. Vol. 19 2004 pp. 1826-183) and 2-chloro-4-{[2-{[(2R)-1-hydroxy-3-methylbutan-2-yl]amino }-9-(propan-2-yl)-9H-purin-6-yl]amino}benzoic acid (purvalanol) (Knockaer et al. Oncogene. Vol. 21 2002 pp 6413-6424.)

Other examples of ERK inhibitors can include PD173074, SC1 (Pluripotin), GW5074, BAY 43-9006, AG 99, CAY10561, ISIS 5132, Apigenin, SP600125, SU4984, SB203580, PD169316, and/or ERK activation inhibitor peptides, AG1478, 3-cyano-4-(phenoxyanilno)quinolines (such as Wyeth-Ayerst Compound 14), a peptide corresponding to the amino-terminal 13 amino acids of MEK1. Still other examples of ERK inhibitors include pyrazole compositions and isoxazole compositions, such as disclosed in U.S. Pat. No 6,495,582,. Yet other examples of ERK inhibitors that may be used include those disclosed in U.S. patent publication 2003/0060469 (Ludwig S. et al.); U.S. patent publication 2004/0048861 (Bemis G. et al.); and U.S. patent publication 2004/0082631 (Hale M. et al.), ERK inhibitor described in Japanese Laid-Open Patent Publication (Tokkai) No. 2005-330265, 5-Iodotubercidin (4-amino-5-iodo-7-(β-D-ribofuranosyl)pyrrolo[2,3-d]-pyrimidine), 3-(2-aminoethyl)-5-[(4-ethoxyphenyl)methylene]-2,4-thiazolidinedione.

Still other examples of inhibitors of ERK are described in U.S. Patent Nos. 7,517,994, 7,732,616, 7,893,065, 6,147,107, 6,319,955, 7,528,166, 6,063,383, 6,703,420, 6,972,298, 7,169,816, 6,696,440, 7,235,537, 7,425,637, 5,525,625, 6,150,401, 6,346,282, 6,949,558, 6,573,044, and 6,512,010,.

The above-listed therapeutic agents, or pharmaceutically acceptable salts thereof, may be formulated into pharmaceutical dosage forms, together with suitable pharmaceutically acceptable carriers, such as diluents, fillers, salts, buffers, stabilizers, solubilizers, etc. The dosage form may contain other pharmaceutically acceptable excipients for modifying conditions such as pH, osmolarity, taste, viscosity, sterility, lipophilicity, solubility etc.

Suitable dosage forms include solid dosage forms, for example, tablets, capsules, powders, dispersible granules, cachets and suppositories, including sustained release and delayed release formulations.

Powders and tablets will generally comprise from 5% to 70% active ingredient. Suitable solid carriers and excipients are generally known in the art and include, e.g., magnesium carbonate, magnesium stearate, talc, sugar, lactose, etc. Tablets, powders, cachets and capsules are all suitable dosage forms for oral administration.

Liquid dosage forms include solutions, suspensions and emulsions. Liquid form preparations may be administered by intravenous, intracerebral, intraperitoneal, parenteral or intramuscular injection or infusion. Sterile injectable formulations may comprise a sterile solution or suspension of the active agent in a non-toxic, pharmaceutically acceptable diluent or solvent. Diluents and solvents can include sterile water, Ringer's solution and isotonic sodium chloride solution, etc. Liquid dosage forms also include solutions or sprays for intranasal administration.

Aerosol preparations for inhalation may include solutions and solids in powder form, which may be combined with a pharmaceutically acceptable carrier, such as an inert compressed gas.

Also encompassed are dosage forms for transdermal administration, including creams, lotions, aerosols and/or emulsions. These dosage forms may be included in transdermal patches of the matrix or reservoir type, which are generally known in the art.

Pharmaceutical preparations may be conveniently prepared in unit dosage form, according to standard procedures of pharmaceutical formulation. The quantity of active compound per unit dose may be varied according to the nature of the active compound and the intended dosage regime. Generally, this will be within the range 0.1 mg to 1000 mg.

The ERK inhibiting compound may be used alone, i.e., as a monotherapy, or in combination one or more of the compounds currently used as approved or off label as treatments for autistic spectrum disorder, as an adjunctive therapy. The one or more currently used compounds that can be used in combination with the ERK inhibiting compound can be selected from the group consisting of an anti-depressant, anti-psychotic, stimulant and other medications, and preferably selected from risperidone (RISPERDAL), aripiprazole (ABILIFY), citalopram (CELEXA), escitalopram (LEXAPRO), (sertraline (ZOLOFT), methylphenidate (RITALIN), atomoxetine (STRATTEA), memantine (NAMENDA) or minocycline (MINOCIN).

The ERK inhibiting compound can be used in the manufacture of a medicament for treating Fragile X syndrome or autism spectrum disorder. A medicament as described herein may include of the adjunctive drugs mentioned above.

In therapeutic use, the active compound may be administered orally, rectally, parenterally or by inhalation (pulmonary delivery). Oral administration is preferred, particularly for administration to pediatric and adolescent patients. Thus, the medicaments described herein may take the form of any of the known pharmaceutical compositions for such methods of administration. The compositions may be formulated in a manner known to those skilled in the art so as to give a controlled release, for example rapid, extended or sustained release, of the active(s) included in the medicament for use herein.

If the ERK inhibiting compound is provided as an adjunctive therapy or as combination therapy to another drug, the ERK inhibiting compound and the other drug may be incorporated as separate dosage forms or may be formulated into a single dosage form. Whether formulated as separate or combined dosage forms, it may be beneficial to facilitate simultaneous, sequential or extended release of the different drugs into the patient's system. Thus, a single dosage form would be formulated for controlled release of the actives by any formulation technique conventionally known in the art, while separate dosage forms may be administered according to a sequential, *i.e.,* staggered, dosage regimen. Also, even if the medicament only comprises a MAPK inhibiting compound as its active compound, it may nevertheless be desired to formulate the medicament for controlled release into the patient, again according to conventional formulation techniques known in the art.

Pharmaceutically acceptable carriers suitable for use in such compositions are well known in the art. The medicaments used in the invention may contain 0.1 to 99% by weight of active compound and typically contain proportions of active compound formulated in accordance with standardised active levels for different patient types and/or different strengths of medicament depending upon the desired dosage regimen

The medicaments can generally be prepared in unit dosage form. A unit dose comprises the one or more active ingredients in an amount of 0.1 to 1000 mg. Excipients used in the preparation of these medicaments may be standard excipients known in the art for this purpose. Appropriate dosage levels may be determined by any suitable method known to one skilled in the art. It will be understood, however, that the specific dose level for any particular patient will depend upon a variety of factors including the activity of the specific compound employed, the age, body weight, general health, sex, diet, time of administration, route of administration, rate of excretion, drug combination and the severity of the disease undergoing treatment.

Conveniently, the medicament described herein, whether comprising an ERK inhibiting compound alone or in combination with other drugs, is provided in the form of a kit that includes the medicament and instructions that the medicament is to be used for the treatment of one or more autistic spectrum disorders and, preferably, autistic disorder, Asperger Syndrome or Pervasive Development Disorder Not Otherwise Specified, or Fragile X syndrome. If the medicament comprises the ERK inhibiting compound and one or more additional drugs in separate dosage forms, the instructions provide details of the dosage regimen to be employed.

The invention is further illustrated by the following example:

### Example 1

Emerging animal studies suggest that deficient control of ERK phosphorylation is of pathological significance in FXS. In this example, we tested the effect of a MEK inhibitor on the behavior of *Fmr1* KO mice, confirming the role of MEK on ERK phosphorylation. However, since regulation of intracellular signaling pathways may differ according to species and tissue, it is important to determine whether ERK phosphorylation is abnormal in brain tissue from patients with FXS and FXTAS. Given the potential significance of this process in elucidating important pathological markers and the development of therapeutic targets for FXS, it is of note that this is the first examination of altered ERK phosphorylation in human brain tissue.

### Methods

### Tissue

Investigation of human ERK pathways was performed in Fragile X syndrome patients aged 9, 20, 22, 23, 62 and 86 years with a diagnosis confirmed by molecular testing (Table 1). Subjects with a premutation of the FMR1 gene at ages 70, 72 and 81 years were also used (Table 1). For autism, pediatric cases aged 9 and 11 years were used. Analyses were performed in neurons and glia in white matter and hippocampus.

**Table 1 Fragile X Syndrome and FXTAS Cases**

| Diagnosis | Age (yr) | Fixed | Frozen | Gender |
|---|---|---|---|---|
| Control | 8 | | Yes | M |
| Control | 9 | Yes | | F |
| Control | 14 | | Yes | M |
| Control | 21 | Yes | | M |
| Control | 32 | Yes | | F |
| Control | 42 | | Yes | M |
| Control | 65 | | Yes | F |
| Control | 68 | Yes | | M |
| Control | 74 | | Yes | M |
| Control | 75 | | Yes | F |
| Control | 81 | | Yes | M |
| | | | | |
| FXS | 10 | Yes | Yes | M |
| FXS | 22 | Yes | | M |
| FXS | 23 | Yes | Yes | M |
| FXS | 63 | Yes | Yes | M |
| FXS | 86 | | Yes | M |
| Premutation | 70 | | Yes | M |
| Premutation | 72 | Yes | Yes | M |
| Premutation | 81 | | Yes | M |

For this example, formalin fixed samples of brain tissue including areas of the hippocampus were obtained from 4 cases of Fragile X and one case of FXTAS from the NICHD Brain and Tissue Bank for Developmental Disorders at the University of Maryland. In addition, age-matched samples from the same source were obtained at the same time. Tissue was paraffin embedded and 6 µm sections cut for immunohistochemical evaluation. Also provided by the University of Maryland was a series of frozen frontal cortical samples from 4 cases of FXS and 3 cases of FXTAS and age-matched control samples. Additional cortical tissue from control individuals was obtained from the University Hospitals of Cleveland using an approved IRB protocol. Table 1 provides more detailed case information for the samples used in this study.

Immunohistochemical studies were performed on the paraffin embedded sections using the peroxidase-anti-peroxidase method (Sternberger et al Proc Natl Acad Sci U S A. Vol. 82 1985 pp 4274-4276). Sections are first deparaffinized through 2 changes of xylene and then rehydrated through a series of graded ethanol solutions from 100% to 50% ethanol. The sections are incubated in 3% H₂O₂ solution for 30 min to reduce any endogenous peroxidase activity. Finally, they are placed in Tris buffered saline (50mM Tris, 150 mM NaCl, pH=7.6). After a 30 min incubation in 10% normal goat serum (NGS) in TBS, primary antibody is applied for 16 hours. After rinsing in 1% NGS, and a subsequent 10 min in 10% NGS, goat anti- mouse or goat anti-rabbit secondary antibody is applied for 30 min. After rinsing as above, mouse or rabbit peroxidase-anti-peroxidase complex is applied for 1 hr. The slides are rinsed 2X in staining jars of Tris buffer (50mM Tris, pH7.6) and development by 3'-3'-diaminobenzidine (Dako). After staining, the sections are rinsed in Tris, dehydrated through ethanols to xylene and mounted with Permount.

For Western blot analysis, grey matter from the frozen cortex samples was homogenized in 1×cell lysis buffer (Cell Signaling) with the addition of protease inhibitor cocktails (Sigma) and PMSF (1 mM). Protein concentration of the total homogenate was measured using the BCA assay (Pierce). Polyacrylamide gel electrophoresis using 10% gels was performed using 30 µg of protein per well, and proteins transferred to Immobilon (Millipore). After blocking in 10% dry milk, primary antibodies were applied for 16 hours at 4°C, then rinsed 5 times in TBS with 0.1% Tween-20 (Sigma). Peroxidase labeled secondary antibodies were applied for 1 hour at RT, blots rinsed in TBS-Tween as before and developed with enhanced chemiluminescence (Millipore) and exposed to film (BioExpress). Antibodies against total ERK (Cell signaling) and dually phosphorylated ERK 42/44 (Cell Signaling) were used. Actin was used as loading control.

### Drug studies

Male *fmr1* knockout mice were bred and tested at 21 days old. All mice were acclimated to the environment, examined, handled, and weighed prior to initiation of the study to assure adequate health and suitability and to minimize non-specific stress associated with manipulation. During the course of the study, 12/12 light/dark cycles were maintained. The room temperature was maintained between 20 and 23°C with a relative humidity maintained around 50%. Chow and water were provided ad libitum for the duration of the study. Each mouse was randomly assigned across treatment groups. The testing was performed during the animals' light cycle phase. SL327 (Sigma Aldrich, 400 mg/kg) was dissolved in 5% Tween80 and saline and administered i.p. at a dose volume of 10 ml/kg 30 min prior to test. Mice were individually placed in a Plexiglas chamber and allowed to explore for 15 sec following which they were exposed to a 125 dB tone for 2 minutes, followed by 1 minute of no sound, and then a further 2 minute tone. The mice were scored based on their response, latency, and seizure intensity:

| | | | |
|---|---|---|---|
| 0: | no response | 3: | clonic-tonic seizures |
| 1. | wild running and jumping | 4: | tonic seizures |
| 2. | clonic seizures | 5: | respiratory arrest |

Animals exhibiting no response were given latency scores of 240 sec.

### Results

In the brain, specific nuclear localization of pERK was noted in cases of FXS and FXTAS. Glial cell and neuronal cell nuclei displayed pERK immunoreactivity in both the white and grey matter, glial staining predominating. Variable numbers of pERK-positive structures were seen in the mutation cases yet pERK was virtually undetected in age-matched control cases (Fig. 2). In some of the older cases diagnosed with FXTAS, a few pyramidal neurons had distinct granulovacuolar degeneration which is prevalent in Alzheimer disease and common in a few neurons in non-demented aged individuals (Fig. 2 inset).

Further, western blot analysis showed that total Erk1/2 was not different between control cases and those with the mutation (Fig. 3). However, phospho-p44/42 Erk1/2, showed significant accumulation in the FXS cases, while the young age-matched controls displayed none (Fig. 2B). In the FXTAS, some cases showed increased levels of phospho-p44/42 Erk1/2, as well as some of the aged individuals without the mutation. The latter change in controls could be related to the development of AD-like pathology present in the older control individuals. Quantification of the specific protein bands showed significantly higher levels of phospho-p44/42 Erk1/2 in cases of FXS (p<0.0001) and in FXTAS (p<0.0001) (Fig. 2).

SL327 treatment resulted in an absence of any audiogenic seizure activity (Fig. 5A). The effects of SL327 on latency to onset of seizure are shown in Fig. 5B. ANOVA found a significant treatment effect. SL327 significantly increased the latency to seizure compared to its vehicle. Since SL327 resulted in a complete absence of seizures in all mice during testing, a latency of 240 sec was given. The effects of SL327 on survival rate are shown in Fig. 5C. All mice receiving SL327 survived. The effects SL327 on mean seizure score are shown in Fig. 5D. Mice treated with SL327 showed a significantly lower seizure score than vehicle-treated mice. In addition to the absence of seizure (score 2, 3, and 4) and respiratory arrest (score 5), mice treated with SL327 showed an absence of wild running and jumping as well (score 1). Accordingly, all mice received a score of `0' for the duration of testing.

### Example 2

This Example shows the potential anticonvulsant properties of perillyl alcohol in the audiogenic seizure test in FMRI knockout mice.

The reference compound MPEP, an mGluR₅ receptor antagonist, and perillyl alcohol (25 and 50 mg/kg) significantly delayed the onset of audiogenic seizures and respiratory arrest, and attenuated overall seizure score.

### MATERIAL AND METHODS

### Animals

Male FMRI knockout mice were bred at PsychoGenies and tested at 21 days old. All mice were acclimated to the environment, examined, handled, and weighed prior to initiation of the study to assure adequate health and suitability and to minimize non-specific stress associated with manipulation. During the course of the study, 12/12 light/dark cycles were maintained. The room temperature was maintained between 20 and 23°C with a relative humidity maintained around 50%. Chow and water were provided *ad libitum* for the duration of the study. Each mouse was randomly assigned across treatment groups. The testing was performed during the animals' light cycle phase

### Drugs

The following compounds were used. All compounds were administered i.p. at a dose volume of 10ml/kg:
- MPEP HCI *(TRC, Lot10B*/*95104;* 30 mg/kg) was dissolved in sterile injectable saline and administered 30 min prior to testing.
- Perillyl alcohol *(Sigma Aldrich, Lot nla;* 25 and 50 mg/kg) was dissolved in soybean oil and administered 60 min prior to testing.
- No clinical signs or toxicity were observed during pretreatment or testing

### Methods: Audiogenic Seizures

Mice were tested at 21 days of age, and pretreated with vehicle or test compound 30 minutes prior to testing. Mice were individually placed in a Plexiglas chamber and allowed to explore for 15 sec following which they were exposed to a 125 dB tone for 2 minutes, followed by 1 minute of no sound, and then a further 2 minute tone. The mice were scored based on their response, latency, and seizure intensity:

| | | | |
|---|---|---|---|
| 0: | no response | 3: | clonic-tonic seizures |
| 1. | wild running and jumping | 4: | tonic seizures |
| 2. | clonic seizures | 5: | respiratory arrest |

Animals exhibiting no response were given latency scores of 240 sec for data analysis purposes.

### Statistical Analysis

Data were analyzed by analysis of variance (ANOVA) followed by post-hoc comparisons with Fisher Tests when appropriate. An effect was considered significant if p < 0.05. Data are presented as the mean ± standard error of the mean.

### RESULTS

### Latency to Seizure

The effects of MPEP and perillyl alcohol on latency to onset of seizure are shown in Fig 6. ANOVA found a significant treatment effect. Post hoc analysis showed that MPEP and perillyl alcohol (25 and 50 mg/kg) increased the latency to seizure compared to respective vehicle.

### Latency to Respiratory Arrest

The effects of MPEP and perillyl alcohol on latency to respiratory arrest (and subsequent death) are shown in Fig. 7. ANOVA found a significant treatment effect. Post hoc analysis found that MPEP and perillyl alcohol (25 and 50 mg/kg) significantly prolonged the latency to respiratory arrest compared to respective vehicle. Mice treated with MPEP (30 mg/kg) showed no respiratory distress during testing and were therefore all given a latency of 240 sec.

### Mean Seizure Score

The effects of MPEP and perillyl alcohol on mean seizure score are shown in Fig 8. ANOVA found a significant treatment effect. Post hoc analysis showed that compared to vehicle, MPEP and perillyl alcohol (25 and 50 mg/kg) significantly decreased seizure score.

## Claims

1. ERK inhibiting compounds singly or in combination that prevent abnormalities in neuronal connectivity; or a pharmaceutically acceptable salt and/or one or more stereoisomers thereof; for use in the treatment of Fragile X syndrome or autism spectrum disorders associated with abnormalities of ERK, wherein the inhibitor of ERK is selected from the group consisting of 2-(2-amino-3-methoxyphenyl)-4H-chromen-4-one, (2Z,3Z)-bis{amino[(2-aminophenyl)sulfanyl]methylidene}butanedinitrile, 5-[(4-bromo-2-chlorophenyl)amino]-4-fluoro-N-(2-hydroxyethoxy)-1-methyl-1 H-benzimidazole-6-carboxamide, 2-[(2-fluoro-4-iodophenyl)amino]-N-(2-hydroxyethoxy)-1,5-dimethyl-6-oxo-1,6-dihydropyridine-3-carboxamide, 2-(4-chloro-2-fluoro-anilino)-N-(2-hydroxyethoxy)-1,5-dimethyl-6-oxo-pyridine-3-carboxamide, 2-[(2-chloro-4-iodophenyl)amino]-N-(cyclopropylmethoxy)-3,4-difluorobenzamide, (3R,4R)-4-(3,4-Dimethoxybenzyl)-3-(4-hydroxy-3-methoxybenzyl)dihydrofuran-2(3H)-one, (2Z)-3-amino-3-[(4-aminophenyl)sulfanyl]-2-[2-(trifluoromethyl)phenyl]prop-2-enenitrile, hypericin, 3-(3-amino-2H-pyrazolo[3,4-c]pyridazin-5-yl)-2-phenyl-3H-pyrazolo[ 1,5-a]pyridin-8-ium, and 2-chloro-4-{[2- {[(2R)-1-hydroxy-3-methylbutan-2-yl]amino}-9-(propan-2-yl)-9H-purin-6-yl]amino}benzoic acid.

2. ERK inhibiting compounds for use according to claim 1, wherein the ERK inhibiting compound is an inhibitor of ERK selected from the group consisting of hypericin, 3-(3-amino-2H-pyrazolo[3,4-c]pyridazin-5-yl)-2-phenyl-3H-pyrazolo[1,5-a]pyridin-8-ium, and 2-chloro-4- {[2- {[(2R)-1-hydroxy-3-methylbutan-2-yl]amino} -9-(propan-2-yl)-9H-purin-6-yl]amino}benzoic acid, or one or more stereoisomers thereof.

3. ERK inhibiting compounds for use according to claims 1 and 2, wherein the ERK inhibiting compound is in the form of one or more of its stereoisomers.

4. ERK inhibiting compounds for use according to claims 1 to 3, wherein the ERK inhibiting compound is included in a medicament in combination with at least one anti-depressant, anti-psychotic, stimulant and other medications

5. ERK inhibiting compounds for use according to claim 4, wherein the ERK compound is included in a medicament in combination with at least one of risperidone, aripiprazole, citalopram, escitalopram, sertraline, methylphenidate, atomoxetine, memantine or minocycline.

6. ERK inhibiting compounds for use according to claims 1 to 5, wherein the compound is comprised in a medicament which is in a pharmaceutically acceptable form selected from the group consisting of tablets, troches, lozenges, aqueous and oily suspensions, dispersible powders and granules, emulsions, hard and soft capsules, syrups and elixirs.

7. ERK inhibiting compounds for use according to claims 1 to 5, wherein the compound is comprised in a medicament which is formulated for extended release into a subject.

8. ERK inhibiting compounds for use according to claims 1 to 7, wherein the autistic spectrum disorder is selected from autistic disorder, Asperger Syndrome or Pervasive Developmental Disorder Not Otherwise Specified.

9. Use of ERK inhibiting compounds singly or in combination that prevent abnormalities in neuronal connectivity; or a pharmaceutically acceptable salt thereof; in the manufacture of a medicament for the treatment of Fragile X syndrome or autism spectrum disorders associated with abnormalities of ERK, wherein the inhibitor of ERK is selected from the group consisting of 2-(2-amino-3-methoxyphenyl)-4H-chromen-4-one, (2Z,3Z)-bis{amino[(2-aminophenyl)sulfanyl]methylidene}butanedinitrile, 5-[(4-bromo-2-chlorophenyl)amino]-4-fluoro-N-(2-hydroxyethoxy)-1-methyl-1H-benzimidazole-6-carboxamide, 2-[(2-fluoro-4-iodophenyl)amino]-N-(2-hydroxyethoxy)-1,5-dimethyl-6-oxo-1,6-dihydropyridine-3-carboxamide, 2-(4-chloro-2-fluoro-anilino)-N-(2-hydroxyethoxy)-1,5-dimethyl-6-oxo-pyridine-3-carboxamide, 2-[(2-chloro-4-iodophenyl)amino]-N-(cyclopropylmethoxy)-3,4-difluorobenzamide, (3R,4R)-4-(3,4-Dimethoxybenzyl)-3-(4-hydroxy-3-methoxybenzyl)dihydrofuran-2(3H)-one, (2Z)-3-amino-3-[(4-aminophenyl)sulfanyl]-2-[2-(trifluoromethyl)phenyl]prop-2-enenitrile, hypericin, 3-(3-amino-2H-pyrazolo[3,4-c]pyridazin-5-yl)-2-phenyl-3H-pyrazolo[ 1,5-a]pyridin-8-ium, and 2-chloro-4- {[2-{[(2R)-1-hydroxy-3-methylbutan-2-yl]amino}-9-(propan-2-yl)-9H-purin-6-yl]amino}benzoic acid.

## Patentansprüche

1. ERK inhibierende Verbindungen einzeln oder in Kombination, die Abnormalitäten bei neuronaler Konnektivität verhindern; oder ein pharmzeutisch verträgliches Salz und/oder ein oder mehrere Stereoisomere davon; zur Verwendung bei der Behandlung von Fragiles-X-Syndrom oder Autismus-Spektrum-Störungen, die mit Abnormalitäten von ERK einhergehen, wobei der Inhibitor von ERK ausgewählt ist aus der Gruppe, bestehend aus 2-(2-Amino-3-methoxyphenyl)-4H-chromen-4-on, (2Z,3Z)-Bis{amino[(2-aminophenyl)-sulfanyl]-methyliden}-butandinitril, 5-[(4-Brom-2-chlorphenyl)-amino]-4-f luor-N- (2-hydroxyethoxy) -1-methyl-1H-benzimidazol-6-carboxamid, 2- [(2-Fluor-4-j odphenyl) -amino] -N- (2-hydroxyethoxy)-1, 5-dimethyl-6-oxo-1, 6-dihydropyridin-3-carboxamid, 2- (4-Chlor-2-fluor-anilino)-N-(2-hydroxyethoxy)-1,5-dimethyl-6-oxo-pyridin-3-carboxamid, 2-[(2-Chlor-4-jodphenyl)-amino]-N-(cyclopropylmethoxy)-3,4-difluorbenzamid, (3R,4R)-4-(3,4-Dimethoxybenzyl)-3-(4-hydroxy-3-methoxybenzyl)-dihydrofuran-2 (3H) -on, (2Z)-3-Amino-3-[(4-aminophenyl)-sulfanyl]-2-[2-(trifluormethyl)-phenyl]-prop-2-ennitril, Hypericin, 3-(3-Amino-2H-pyrazolo [3,4-c]pyridazin-5-yl)-2-phenyl-3H-pyrazolo [1, 5-a]pyridin-8-ium und 2-Chlor-4- [2-{[(2R)-1-hydroxy-3-methylbutan-2-yl]-amino}-9-(propan-2-yl)-9H-purin-6-yl]-amino}-benzoesäure.

2. ERK inhibierende Verbindungen zur Verwendung nach Anspruch 1, wobei die ERK inhibierende Verbindung ein Inhibitor von ERK ist, ausgewählt aus der Gruppe, bestehend aus Hypericin, 3-(3-Amino-2H-pyrazolo[3,4-c] pyridazin-5-yl)-2-phenyl-3H-pyrazolo[1,5-a]pyridin-8-ium und 2-Chlor-4-{[2-{[(2R)-1-hydroxy-3-methylbutan-2-yl]-amine}-9-(propan-2-yl)-9H-purin-6-yl]-amine}-benzoesäure oder einem oder mehreren Stereoisomeren davon.

3. ERK inhibierende Verbindungen zur Verwendung nach Ansprüchen 1 und 2, wobei die ERK inhibierende Verbindung in Form von einem oder mehreren ihrer Stereoisomere vorliegt.

4. ERK inhibierende Verbindungen zur Verwendung nach Ansprüchen 1 bis 3, wobei die ERK inhibierende Verbindung in einem Medikament in Kombination mit mindestens einem Antidepressivum, Antipsychotikum, Stimulans und anderen Medikationen enthalten ist.

5. ERK inhibierende Verbindungen zur Verwendung nach Anspruch 4, wobei die ERK-Verbindung in ein Medikament in Kombination mit mindestens einem von Risperidon, Aripiprazol, Citalopram, Escitalopram, Sertralin, Methylphenidat, Atomoxetin, Memantin oder Minocyclin enthalten ist.

6. ERK inhibierende Verbindungen zur Verwendung nach Ansprüchen 1 bis 5, wobei die Verbindung in einem Medikament enthalten ist, welches in einer pharmazeutisch verträglichen Form, ausgewählt aus der Gruppe, bestehend aus Tabletten, Pastillen, Dragees, wässrigen und öligen Suspensionen, dispergierbaren Pulvern und Granulaten, Emulsionen, Hart- und Weich-Kapseln, Sirupen und Elixieren, vorliegt.

7. ERK inhibierende Verbindungen zur Verwendung nach Ansprüchen 1 bis 5, wobei die Verbindung in einem Medikament enthalten ist, welches zur längeren Freisetzung in einer Person formuliert ist.

8. ERK inhibierende Verbindungen zur Verwendung nach Ansprüchen 1 bis 7, wobei die Autismus-Spektrum-Störung ausgewählt ist aus autistischer Störung, Asperger-Syndrom oder tiefgreifenden Entwicklungsstörungen - nicht anders bezeichnet (PDD-NOS).

9. Verwendung von ERK inhibierenden Verbindungen einzeln oder in Kombination, die Abnormalitäten in der neuronalen Konnektivität verhindern; oder einem pharmazeutisch verträglichen Salz davon; bei der Herstellung eines Medikaments für die Behandlung von Fragiles-X-Syndrom oder Autismus-Spektrum-Störungen, verbunden mit Abnormalitäten von ERK, wobei der Inhibitor von ERK ausgewählt ist aus der Gruppe, bestehend aus 2-(2-Amino-3-methoxyphenyl) -4H-chromen-4-on, (2Z,3Z)-Bis{amino-[(2-aminophenyl)-sulfanyl]-methyliden}-butandinitril, 5-[(4-Brom-2-chlorphenyl)-amino]-4-f luor-N- (2-hydroxyethoxy) -1-methyl-1H-benz imidazol-6-carboxamid, 2-[(2-Fluor-4-jodphenyl)-amino]-N-(2-hydroxyethoxy)-1,5-dimethyl-6-oxo-1,6-dihydropyridin-3-carboxamid, 2-(4-Chlor-2-fluor-anilino)-N-(2-hydroxyethoxy) -1, 5-dimethyl-6-oxo-pyridin-3-carboxamid, 2-[(2-Chlor-4-jodphenyl)-amino]-N-(cyclopropylmethoxy)-3,4-difluorbenzamid, (3R,4R)-4-(3,4-Dimethoxybenzyl)-3-(4-hydroxy-3-methoxybenzyl) -dihydrofuran-2 (3H)-on, (2Z)-3-Amino-3-[(4-aminophenyl)-sulfanyl]-2-[2-(trifluormethyl)-phenyl]-prop-2-ennitril, Hypericin, 3- (3-Amino-2H-pyrazolo [3,4-c] pyridazin-5-yl) -2-phenyl-3H-pyrazolo[1,5-a]pyridin-8-iumund2-Chlor-4-{[2-{[(2R)-1-hydroxy-3-methylbutan-2-yl]amino}-9-(propan-2-yl)-9H-purin-6-yl]-amino}-benzoesäure.

## Revendications

1. Composés inhibiteurs d'ERK seuls ou en combinaison, qui préviennent des anomalies de la connectivité neuronale ; ou un sel pharmaceutiquement acceptable et/ou un ou plusieurs stéréoisomère(s) de ceux-ci ; pour utilisation dans le traitement du syndrome X fragile ou de troubles du spectre de l'autisme associés à des anomalies de l'ERK, dans lesquels l'inhibiteur d'ERK est choisi dans l'ensemble constitué par la 2-(2-amino-3-méthoxyphényl)-4H-chromén-4-one, le (2Z,3Z)-bis{amino[(2-aminophényl)sulfanyl]méthylidène}butanedinitrile, le 5-[(4-bromo-2-chlorophényl)amino]-4-fluoro-N-(2-hydroxyéthoxy)-1-méthyl-1H-benzimidazole-6-carboxamide, le 2-[(2-fluoro-4-iodophényl)amino]-N-(2-hydroxyéthoxy)-1,5-diméthyl-6-oxo-1,6-dihydropyridine-3-carboxamide, le 2-(4-chloro-2-fluoroanilino)-N-(2-hydroxyéthoxy)-1,5-diméthyl-6-oxopyridine-3-carboxamide, le 2-[(2-chloro-4-iodophényl)amino]-N-(cyclopropylméthoxy)-3,4-difluorobenzamide, le (3R,4R)-4-(3,4-diméthoxybenzyl)-3-(4-hydroxy-3-méthoxybenzyl)dihydrofuran-2(3H)-one, le (2Z)-3-amino-3-[(4-aminophényl)sulfanyl]-2-[2-(trifluorométhyl)phényl]prop-2-ènenitrile, l'hypéricine, le 3-(3-amino-2H-pyrazolo[3,4-c]pyridazin-5-yl)-2-phényl-3H-pyrazolo[1,5-a]pyridin-8-ium, et l'acide 2-chloro-4-{[2-{[(2R)-1-hydroxy-(3-méthylbutan-2-yl]amino}-9-(propan-2-yl)-9H-purin-6-yl]amino}benzoïque.

2. Composés inhibiteurs d'ERK pour utilisation selon la revendication 1, dans lesquels le composé inhibiteur d'ERK est un inhibiteur d'ERK choisi dans l'ensemble constitué par l'hypéricine, le 3-(3-amino-2H-pyrazolo[3,4-c]pyridazin-5-yl)-2-phényl-3H-pyrazolo[1,5-a]pyridin-8-ium, et l'acide 2-chloro-4-{[2-{[(2R)-1-hydroxy-(3-méthylbutan-2-yl]amino}-9-(propan-2-yl)-9H-purin-6-yl]amino}benzoïque, ou un ou plusieurs stéréoisomère(s) de celui-ci.

3. Composés inhibiteurs d'ERK pour utilisation selon les revendications 1 et 2, dans lesquels le composé inhibiteur d'ERK est sous la forme d'un ou plusieurs de ses stéréoisomères.

4. Composés inhibiteurs d'ERK pour utilisation selon les revendications 1 à 3, dans lesquels le composé inhibiteur d'ERK est incorporé dans un médicament en combinaison avec au moins un antidépresseur, un antipsychotique, un stimulant, et d'autres médicaments.

5. Composés inhibiteurs d'ERK pour utilisation selon la revendication 4, dans lesquels le composé inhibiteur d'ERK est incorporé dans un médicament en combinaison avec au moins l'un parmi la rispéridone, l'aripiprazole, le citalopram, l'escitalopram, la sertraline, le méthylphénidate, l'atomoxétine, la mémantine et la minocycline.

6. Composés inhibiteurs d'ERK pour utilisation selon les revendications 1 à 5, dans lesquels le composé est incorporé dans un médicament qui est sous une forme pharmaceutiquement acceptable choisie dans l'ensemble constitué par les comprimés, les tablettes, les pastilles les suspensions aqueuses et huileuses, les poudres et granules dispersibles, les émulsions, les capsules dures et molles, les sirops, et les élixirs.

7. Composés inhibiteurs d'ERK pour utilisation selon les revendications 1 à 5, dans lesquels le composé est incorporé dans un médicament qui est formulé pour une libération prolongée chez un sujet.

8. Composés inhibiteurs d'ERK pour utilisation selon les revendications 1 à 7, dans lesquels le trouble du spectre autistique est choisi parmi le trouble autistique, le syndrome d'Asperger et le trouble envahissant du développement non spécifié.

9. Utilisation de composés inhibiteurs d'ERK seuls ou en combinaison qui préviennent des anomalies de la connectivité neuronale ; ou d'un sel pharmaceutiquement acceptable de tels composés ; dans la fabrication d'un médicament destiné au traitement du syndrome X fragile ou de troubles du spectre de l'autisme associés à des anomalies de l'ERK, dans laquelle l'inhibiteur d'ERK est choisi dans l'ensemble constitué par la 2-(2-amino-3-méthoxyphényl)-4H-chromén-4-one, le (2Z,3Z)-bis{amino[(2-aminophényl)sulfanyl]méthylidène}butanedinitrile, le 5-[(4-bromo-2-chlorophényl)amino]-4-fluoro-N-(2-hydroxyéthoxy)-1-méthyl-1H-benzimidazole-6-carboxamide, le 2-[(2-fluoro-4-iodophényl)amino]-N-(2-hydroxyéthoxy)-1,5-diméthyl-6-oxo-1,6-dihydropyridine-3-carboxamide, le 2-(4-chloro-2-fluoroanilino)-N-(2-hydroxyéthoxy)-1,5-diméthyl-6-oxopyridine-3-carboxamide, le 2-[(2-chloro-4-iodophényl)amino]-N-(cyclopropylméthoxy)-3,4-difluorobenzamide, le (3R,4R)-4-(3,4-diméthoxybenzyl)-3-(4-hydroxy-3-méthoxybenzyl)dihydrofuran-2(3H)-one, le (2Z)-3-amino-3-[(4-aminophényl)sulfanyl]-2-[2-(trifluorométhyl)phényl]prop-2-ènenitrile, l'hypéricine, le 3-(3-amino-2H-pyrazolo[3,4-c]pyridazin-5-yl)-2-phényl-3H-pyrazolo[1,5-a]pyridin-8-ium, et l'acide 2-chloro-4-{[2-{[(2R)-1-hydroxy-(3-méthylbutan-2-yl]amino}-9-(propan-2-yl)-9H-purin-6-yl]amino}benzoïque.
